# EUROPEAN PATENT SPECIFICATION

(11) **EP 4 444 693 B1**
(45) Date of publication and mention of the grant of the patent: **18.02.2026**
(21) Application number: 22826713.4
(22) Date of filing: 05.12.2022
(51) Int. Cl.: C07C 231/02, C07C 233/15

(54) **A PROCESS FOR PREPARING N-SUBSTITUTED ACETAMIDE**
VERFAHREN ZUR HERSTELLUNG VON N-SUBSTITUIERTEM ACETAMID
PROCÉDÉ DE PRÉPARATION D'ACÉTAMIDE N-SUBSTITUÉ

(30) Priority: 06.12.2021 US 202163286108 P
(43) Date of publication of application: 16.10.2024
(73) Proprietor: Adama Agan Ltd., 7710001 Ashdod (IL)
(72) Inventor: TZOR, Omer, 5565406 Kiryat-Ono (IL)
(74) Representative: Modiano, Gabriella Diana
(86) International application number: PCT/IL2022/051288
(87) International publication number: WO 2023/105516

(56) References cited:
- CN-A- 105 017 067
- YU CHUANMING, ET AL.: "Synthesis of fluorochloridone", NONGYAO, vol. 40, no. 7, 2001, CN, pages 18 - 19, XP093033909, ISSN: 1006-0413
- YANG JIANBO, ET AL.: "Synthesis of herbicide fluorochloridone", JINGXI HUAGONG ZHONGJIANTI, vol. 35, no. 4, August 2005 (2005-08-01), CN, pages 24 - 27, XP093033903, ISSN: 1009-9212

## Description

### FIELD OF THE INVENTION

The present invention relates to a process for preparing N-substituted acetamides by condensing aniline or derivative thereof with chlorinated acyl chloride.

### BACKGROUND

N-substituted acetamide serves as a key intermediate for synthesis of pyrrolidinone class of pesticides (such as Flurochloridone having IUPAC name 3-chloro-4-chloromethyl-1-[3-(trifluoromethyl) phenyl]pyrrolidin-2-one).

While numerous methods have been reported so far for preparation of N-substituted acetamides, condensation of aniline or derivatives thereof with an acyl chloride under different reaction conditions using catalytic agents/promoters is generally preferred.

Conventional wet method for synthesis of N-substituted acetamide involves addition of an acyl chloride to a biphasic system containing aniline or derivatives thereof, a water immiscible solvent, water and a base, at low temperature under slightly basic to slightly acidic pH. However, this process suffers from several disadvantages such as: (i) higher consumption of acyl chloride (20-50% molar excess over aniline or derivative thereof), due to hydrolysis of acyl chloride in presence of water; (ii) generation of additional by-product such as carboxylic acid in the reaction mixture; and (iii) increase in consumption of base, as the hydrolysis of acyl chloride results in production of HCl as by-product, which needs to be neutralized. Formation of carboxylic acid due to hydrolysis of acyl chloride is schematically represented below:

RCOC1 + H₂O **→** RCOOH + HCl

Conventional dry method for preparation of N-substituted acetamide, involves addition of an acyl chloride to a monophasic system containing aniline or derivatives thereof and an organic solvent at a reflux temperature. Although this approach affords the advantages of (i) lower consumption of acyl chloride (10-15% molar excess over aniline or derivatives thereof), since no hydrolysis take place in absence of water and (ii) no requirement of base, this process is not widely used at an industrial scale owing to major safety issues over the emission of hydrogen chloride gas. Emitted hydrogen chloride may be treated in 2 ways: (a) continuous emission into an alkaline solution and neutralization into a chloride salt of the alkaline base, the salt may be further utilized; and (b) continuous absorption into an aqueous solution to form a hydrochloric acid solution, which may be further utilized.

Yu Chuanming, et al. (Nongyao, 2001, 40(7), 18-19) and Yang Jinnbao, et al. (Jingxi Huagong Zhongjianti (Fine Chemical Intermediates) 2005, 35(4), 24-27) disclose methods for preparing N-allyl-2,2-dichloro-N-(3-trifluoromethylphenyl)acetamide by the reaction of N-allyl-3-trifluoromethylaniline with dichloroacetyl chloride in various solvents and also with no solvent.

Further known processes for synthesis of N-substituted acetamide are likewise not completely satisfactory with regard to consumption of acyl chloride, amount of by-product obtained and the likes. Accordingly, need is felt of a new and improved process for synthesis of N-substituted acetamide that may overcome one or more problems associated with the conventional processes. The present invention satisfies the existing needs, as well as others, and generally overcomes the deficiencies found in the state of art.

### OBJECTS

Primary object of the present invention is to provide a new and improved process that may overcome one or more limitations associated with the conventional processes for synthesis of N-substituted acetamide.

It is an object of the present disclosure to provide an improved process for preparation of N-substituted acetamide that reduces the amount of by-product produced during the reaction.

Another object of the present disclosure is to provide a process for synthesis of N-substituted acetamide that decreases consumption of the reactant(s).

It is also an object of the present disclosure to provide a process for synthesis of N-substituted acetamide that is easily to follow, economical and industrially applicable.

It is also an object of the present disclosure to provide a process for synthesis of N-allyl-2,2-dichloro-N-(3-(trifluoromethyl)phenyl)acetamide that is easily to follow, economical and industrially applicable.

### SUMMARY

The present invention relates to a process for preparing N-substituted acetamide by condensing aniline or derivative thereof with a chlorinated acyl chloride.

The present disclosure is on the premise of a surprising discovery by inventors of the present disclosure that - during the course of condensation reaction between a chlorinated acyl chloride and aniline or derivative thereof, in presence of a mixture of a water immiscible solvent and water, hydrolysis of chlorinated acyl chloride constantly competes with condensation thereof with aniline or derivative thereof. At the lower conversion rates (such as, at the initial stage of the condensation reaction) the rate of hydrolysis is negligible relative to the rate of condensation, but when the higher conversion is achieved (such as, when more than about 60-70% aniline or derivative thereof has been converted to N-substituted acetamide), the hydrolysis rate becomes significant and rate limiting. At such higher conversion rates, significant amounts of chlorinated acyl chloride, present in or being added to the reaction mixture, gets converted to carboxylic acid requiring addition of at least 20% molar excess of chlorinated acyl chloride for completion of the reaction. It could also be noted, albeit surprisingly, that when higher conversion rates are achieved (i.e. when more than about 60-70%, particularly, 80-90% aniline or derivative thereof has been converted to N-substituted acetamide), the reaction, if switched to dry mode, by substantially removing aqueous phase from the reaction mixture (for example, by discarding the aqueous phase, followed by heating at reflux temperature, optionally, with azeotropic distillation), precludes the competing hydrolysis reaction, significantly reducing the total amount of chlorinated acyl chloride required for synthesis of N-substituted acetamide, while also reducing the risk of accumulation of HCl gas in comparison to a process, which is operated in dry mode only. Without wishing to be bound by the theory, it is believed that as the ratio of water to organic phase is higher, the surface of the interphase will be higher and the hydrolysis rate will be higher as well. For this reason, as the amount of water will be lower, the consumption of chlorinated acyl chloride will be lower. Nevertheless, a certain amount of water is required to allow efficient hydrogen chloride neutralization and to allow pH monitoring (where applicable) as well.

Accordingly, an aspect of the present disclosure relates to a process for preparing a N-substituted acetamide, the process comprising the steps of: (a) reacting an aniline or derivative thereof with a first amount of a chlorinated acyl chloride in presence of a mixture of an organic solvent and water for a first time period; (b) discarding the aqueous phase from the reaction mixture; (c) heating the reaction mixture at a reflux temperature; (d) effecting addition of a second amount of the chlorinated acyl chloride to the refluxing reaction mixture; and (e) continuing the reaction at the reflux temperature for a second time period to obtain a crude product mixture comprising N-substituted acetamide.

In an embodiment, the N-substituted acetamide is N-allyl-2,2-dichloro-N-(3-(trifluoromethyl) phenyl)acetamide, the chlorinated acyl chloride is 2,2-dichloroacetyl chloride, and the substituted aniline is N-allyl-3-(trifluoromethyl) aniline.

Other aspects, advantages, and salient features of the invention will become apparent to those skilled in the art from the following detailed description, which, taken in conjunction with the exemplary embodiments of the invention.

### DETAILED DESCRIPTION

The following is a detailed description of embodiments of the present invention. The embodiments are in such detail as to clearly communicate the invention.

Groupings of alternative elements or embodiments of the invention disclosed herein are not to be construed as limitations. Each group member can be referred to and claimed individually or in any combination with other members of the group or other elements found herein. One or more members of a group can be included in, or deleted from, a group for reasons of convenience and/or patentability.

Unless the context requires otherwise, throughout the specification which follow, the word "comprise" and variations thereof, such as, "comprises" and "comprising" are to be construed in an open, inclusive sense that is as "including, but not limited to."

Reference throughout this specification to "one embodiment" or "an embodiment" means that a particular feature, structure or characteristic described in connection with the embodiment is included in at least one embodiment. Thus, the appearances of the phrases "in one embodiment" or "in an embodiment" in various places throughout this specification are not necessarily all referring to the same embodiment. Furthermore, the particular features, structures, or characteristics may be combined in any suitable manner in one or more embodiments.

As used in the description herein and throughout the claims that follow, the meaning of "a," "an," and "the" includes plural reference unless the context clearly dictates otherwise. Also, as used in the description herein, the meaning of "in" includes "in" and "on" unless the context clearly dictates otherwise.

In some embodiments, the numbers expressing quantities of ingredients, properties such as concentration, and so forth, used to describe and claim certain embodiments of the invention are to be understood as being modified in some instances by the term "about."Accordingly, in some embodiments, the numerical parameters set forth in the written description are approximations that can vary depending upon the desired properties sought to be obtained by a particular embodiment. In some embodiments, the numerical parameters should be construed in light of the number of reported significant digits and by applying ordinary rounding techniques. Notwithstanding that the numerical ranges and parameters setting forth the broad scope of some embodiments of the invention are approximations, the numerical values set forth in the specific examples are reported as precisely as practicable.

The recitation of ranges of values herein is merely intended to serve as a shorthand method of referring individually to each separate value falling within the range. Unless otherwise indicated herein, each individual value is incorporated into the specification as if it were individually recited herein.

The headings and abstract of the invention provided herein are for convenience only and do not interpret the scope or meaning of the embodiments.

All methods described herein can be performed in any suitable order unless otherwise indicated herein or otherwise clearly contradicted by context. The use of any and all examples, or exemplary language (e.g. "such as") provided with respect to certain embodiments herein is intended merely to better illuminate the invention and does not pose a limitation on the scope of the invention otherwise claimed. No language in the specification should be construed as indicating any non-claimed element essential to the practice of the invention.

The present invention relates to a process for preparing N-substituted acetamide by condensing aniline or derivative thereof with chlorinated acyl chloride.

An aspect of the present disclosure relates to a process for preparing a N-substituted acetamide, the process comprising the steps of: (a) reacting an aniline or derivative thereof with a first amount of a chlorinated acyl chloride in presence of a mixture of an organic solvent and water for a first time period; (b) discarding the aqueous phase from the reaction mixture; (c) heating the reaction mixture at a reflux temperature; (d) effecting addition of a second amount of the chlorinated acyl chloride to the refluxing reaction mixture; and (e) continuing the reaction at the reflux temperature for a second time period to obtain a crude product mixture comprising N-substituted acetamide.

Aniline or derivative thereof is reacted with the first amount of chlorinated acyl chloride in presence of a mixture of an organic solvent and water for a first time period such that about 50-95% of aniline or derivative thereof gets converted to N-substituted acetamide. Accordingly, the first amount of chlorinated acyl chloride may be selected such that molar ratio between Aniline or derivative thereof and chlorinated acyl chloride ranges from 1:0.5 to 1:1.1. Preferably, the first amount is selected such that about 75-95% of aniline or derivative thereof gets converted to N-substituted acetamide. In an embodiment, the first amount of chlorinated acyl chloride is selected such that molar ratio between aniline or derivative thereof and chlorinated acyl chloride ranges from 1:0.8 to 1:1.1.

After completion of reaction between aniline or derivative thereof and the first amount of chlorinated acyl chloride, the aqueous phase from the reaction mixture is discarded, and the reaction mixture is heated to a reflux temperature, optionally, with azeotropic distillation. The reflux temperature may range from 80°C to 200°C, depending on the boiling point of the solvent.

A second amount of chlorinated acyl chloride is then added to the refluxing reaction mixture to drive the condensation reaction to completion. Accordingly, the second amount of chlorinated acyl chloride may be selected such that molar ratio between aniline or derivative thereof (initially charged) and the second amount of chlorinated acyl chloride ranges from 1:0.05 to 1:0.5. One would appreciate that since only ~10-20% of total amount of chlorinated acyl chloride is reacted in the dry mode, the risk of hydrogen chloride accumulation is significantly reduced in the advantageous process of the present disclosure.

Accordingly, the advantageous process of the present disclosure for preparation of N-substituted acetamide involves switching from the wet mode to the dry mode by substantially removing aqueous phase from the reaction mixture (for example, by discarding the aqueous phase, followed by heating at reflux temperature, optionally with azeotropic distillation), precluding the competing hydrolysis reaction during the phase where the rate of hydrolysis is relatively higher (i.e. when more than about 60-70%, particularly, 80-90% of aniline or derivative thereof has been converted to N-substituted acetamide). This affords dramatic reduction in the total amount of chlorinated acyl chloride required for the preparation of N-substituted acetamide, while retaining the advantage of reduced production of HCl gas.

While the advantageous process of the present disclosure can be used for synthesis of wide verities of N-substituted acetamides, in an exemplary embodiment, the process is implemented for the synthesis of N-allyl-2,2-dichloro-N-(3-(trifluoromethyl)phenyl)acetamide, which serves as a key intermediate for synthesis of Flurochloridone.

A general schema showing condensation reaction between N-allyl-3-(trifluoromethyl)aniline and 2,2-dichloroacetyl chloride for preparation of N-allyl-2,2-dichloro-N-(3-(trifluoromethyl)phenyl) acetamide by condensing is provided below:

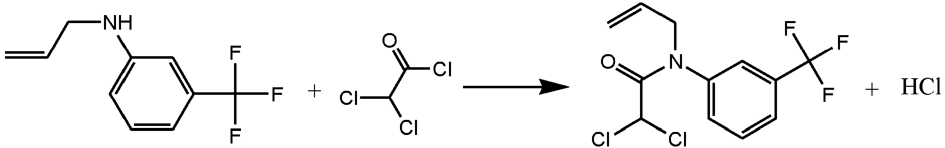

A process for preparing N-allyl-2,2-dichloro-N-(3-(trifluoromethyl)phenyl) acetamide includes the steps of: (a) reacting N-allyl-3-(trifluoromethyl) aniline with a first amount of 2,2-dichloroacetyl chloride in presence of a mixture of an organic solvent and water for a first time period; (b) discarding the aqueous phase from the reaction mixture; (c) heating the reaction mixture at a reflux temperature, optionally with azeotropic distillation; (d) effecting addition of a second amount of 2,2-dichloroacetyl chloride to the refluxing reaction mixture; and (e) continuing the reaction at the reflux temperature for a second time period to obtain a crude product mixture comprising N-allyl-2,2-dichloro-N-(3-(trifluoromethyl)phenyl)acetamide.

In an embodiment, the process includes the steps of: (a) reacting N-allyl-3-(trifluoromethyl) aniline with a first amount of 2,2-dichloroacetyl chloride in presence of a mixture of an organic solvent and water under an inert atmosphere at a temperature ranging from 5°C to 30°C maintaining a pH between 5.0 and 11.0 for a first time period; (b) discarding the aqueous phase from the reaction mixture; (c) heating the reaction mixture to a reflux temperature, optionally, with azeotropic distillation; (d) effecting addition of a second amount of 2,2-dichloroacetyl chloride to the refluxing reaction mixture; and (e) continuing the reaction at the reflux temperature for a second time period to obtain a crude product mixture comprising N-allyl-2,2-dichloro-N-(3-(trifluoromethyl)phenyl)acetamide.

In an embodiment, pH is maintained between 5.0 and 11.0 by effecting addition of a buffering agent/compound before and/or during the course of reaction between N-allyl-3-(trifluoromethyl) aniline with a first amount of 2,2-dichloroacetyl chloride. Alternatively or in addition, pH of the reaction mixture may be continuously or intermittently monitored and maintained between 5.0 and 11.0 by effecting addition of a base. Still alternatively, pH of the reaction mixture may be continuously or intermittently monitored and maintained between 5.0 and 11.0 by addition of a catalytic amount of the buffering agent/compound affording reduction in consumption of the buffering agent, improvement in pH control and enhanced safety in case of failure in pH control.

In an embodiment, the step of reacting N-allyl-3-(trifluoromethyl) aniline with the first amount of 2,2-dichloroacetyl chloride comprises reacting N-allyl-3-(trifluoromethyl) aniline with the first amount of 2,2-dichloroacetyl chloride in a molar ratio ranging from 1:0.5 to 1:1.1. Alternatively, N-allyl-3-(trifluoromethyl) aniline is reacted with the first amount of 2,2-dichloroacetyl chloride in a molar ratio ranging from 1:0.7 to 1:1.1. Preferably, N-allyl-3-(trifluoromethyl) aniline is reacted with the first amount of 2,2-dichloroacetyl chloride in a molar ratio ranging from 1:0.8 to 1:1.1.

In an embodiment, the step of reacting N-allyl-3-(trifluoromethyl)aniline with 2,2-dichloroacetyl chloride comprises: (i) charging N-allyl-3-(trifluoromethyl)aniline and the mixture of organic solvent and water in a reaction vessel under the inert atmosphere to prepare a solution; (ii) cooling the solution to a temperature ranging from 5°C to 20°C; (iii) effecting addition of the first amount of 2,2-dichloroacetyl chloride to the cooled solution over a time period ranging from 60 minutes to 240 minutes maintaining temperature within 5°C and 20°C and pH within 5.0 to 11.0; and (iv) stirring the reaction mixture for a time period ranging from 30 minutes to 120 minutes.

The organic solvent preferably is a water immiscible solvent and inert to 2,2-dichloroacetyl chloride. Non-limiting examples of the organic solvents that may be useful in the process of the present disclosure includes, but not limited to, benzene, toluene, carbontetrachloride, cyclohexane, dichloromethane, ethyl acetate, 1,2-dichloroethane, chlorobenzene, 1,2-dichlorobenzene, 1,3-dichlorobenzene, xylene, butanone, methylisobutyl ketone, methyl tert-butyl ether, chloroform, diethylether and mixtures thereof.

The first time period may range from 90 minutes to 360 minutes.

In an embodiment, the step of effecting addition of the second amount of 2,2-dichloroacetyl chloride to the refluxing reaction mixture comprises adding 2,2-dichloroacetyl chloride in an amount such that the molar ratio of N-allyl-3-(trifluoromethyl) aniline (initially charged amount) to the second amount of 2,2-dichloroacetyl chloride ranges from 1:0.05 to 1:0.5.

In an embodiment, the step of effecting addition of the second amount of 2,2-dichloroacetyl chloride to the refluxing reaction mixture comprises adding the second amount of 2,2-dichloroacetyl chloride to the refluxing reaction mixture over a time period ranging from 15 minutes to 120 minutes.

The reflux temperature may range from 80°C to 200°C, depending on the boiling point of the organic solvent employed. In an embodiment, the reflux temperature range from 80°C to 150°C.

The second time period may range from 30 minutes to 120 minutes.

In an embodiment, the process includes the steps of: (a) reacting N-allyl-3-(trifluoromethyl) aniline with a first amount of 2,2-dichloroacetyl chloride in presence of a mixture of an organic solvent and water under an inert atmosphere at a temperature ranging from 5°C to 30°C maintaining a pH between 5.0 and 11.0 for a time period ranging from 30 minutes to 360 minutes; (b) discarding the aqueous phase from the reaction mixture, (c) heating the reaction mixture to a reflux temperature, optionally, with azeotropic distillation; (d) effecting addition of a second amount of 2,2-dichloroacetyl chloride to the refluxing reaction mixture; and (e) continuing the reaction at the reflux temperature for a time period ranging from 30 minutes to 120 minutes to obtain a crude product mixture comprising N-allyl-2,2-dichloro-N-(3-(trifluoromethyl)phenyl)acetamide.

In an embodiment, the process is for the preparation of Flurochloridone, the process comprising preparing an N-substituted acetamide as previously described.

In an embodiment, the process for the preparation, of the an N-substituted acetamide as an intermediate in the synthesis of Flurochloridone.

The foregoing description of the specific embodiments will so fully reveal the general nature of the embodiments herein that others can, by applying current knowledge, readily modify and/or adapt for various applications such specific embodiments without departing from the generic concept, and, therefore, such adaptations and modifications should and are intended to be comprehended within the meaning of the disclosed embodiments. It is to be understood that the phraseology or terminology employed herein is for the purpose of description and not of limitation. Therefore, while the embodiments herein have been described in terms of preferred embodiments, those skilled in the art will recognize that the embodiments herein can be practiced with modification within the scope of the embodiments as described herein.

### EXAMPLES

The disclosure will now be illustrated with working examples, which is intended to illustrate the working of disclosure and not intended to take restrictively to imply any limitations on the scope of the present disclosure. Unless defined otherwise, all technical and scientific terms used herein have the same meaning as commonly understood to one of ordinary skill in the art to which this disclosure belongs. Although methods and materials similar or equivalent to those described herein can be used in the practice of the disclosed methods and compositions, the exemplary methods, devices and materials are described herein. It is to be understood that this disclosure is not limited to particular methods, and experimental conditions described, as such methods and conditions may vary.

### Preparation of N-allyl-2,2-dichloro-N-(3-(trifluoromethyl)phenyl)acetamide

About 200 gm of N-allyl-3-(trifluoromethyl)aniline, 357 gm of toluene and 57 gm of water were charged in a reaction vessel under nitrogen atmosphere. The mixture was cooled to about 15°C. About 139.2 gm of 2,2-dichloroacetyl chloride was then added to the above cooled mixture over a time period of about 3 hours maintaining temperature at about 15°C and pH between 5.0 to 7.0 using NaOH (30% in water). The reaction mixture was stirred for about 1 hr at 15°C. The aqueous phase from the reaction mixture was then discarded and the organic phase (i.e. remainder of the reaction mixture) was then heated to a reflux temperature of about 120°C. 23.0 gm of 2,2-dichloroacetyl chloride was then added over 1 hour time in the refluxing reaction mixture, and further refluxed for about 1 hour under constant stirring. The reaction mixture was then cooled to about 25°C to obtain crude product mixture including N-allyl-2,2-dichloro-N-(3-(trifluoromethyl)phenyl)acetamide.

The crude product mixture was then mixed with 130 gm of water followed by neutralization (between pH 6.0-7.0) with NaOH (30% in water). Again the aqueous phase was discarded and the remaining organic phase was mixed with about 57 gm of water. The aqueous phase was again discarded and the remaining organic phase was heated to evaporate any water present in the organic phase to obtain 305.1 gm of N-allyl-2,2-dichloro-N-(3-(trifluoromethyl)phenyl)acetamide.

Based on the experiments, it could be concluded that the advantageous process of the present disclosure affords (i) reduction in consumption of 2,2-dichloroacetyl chloride by about 5-15% in comparison to conventional wet method while affording similar yields of N-allyl-2,2-dichloro-N-(3-(trifluoromethyl)phenyl)acetamide; (ii) reduction in consumption of base by about 30-40%; and (iii) reduction in emission of HCl gas by about 60-90% in comparison to conventional dry methods greatly improving the process safety, while affording significant economic benefits.

It will be evident to those skilled in the art that the invention is not limited to the details of the foregoing illustrative examples and that the present invention may be embodied in other specific forms without departing from the essential attributes thereof. It is therefore desired that the present embodiments and examples be considered in all respects as illustrative and not restrictive, reference being made to the appended claims, rather than to the foregoing description and all the changes which come within the meaning and range of equivalency of the claims are therefore intended to be embraced therein.

### ADVANTAGES

The present disclosure provides a new and improved process that may overcome one or more limitations associated with the conventional processes for synthesis of N-substituted acetamide.

The present disclosure provides an improved process for preparation of N-substituted acetamide that reduces the amount of by-product produced during the reaction.

The present disclosure provides a process for synthesis of N-substituted acetamide that decreases consumption of the reactant(s).

The present disclosure provides a process for synthesis of N-substituted acetamide that is easily to follow, economical and industrially applicable.

The present disclosure provides a process for synthesis of N-allyl-2,2-dichloro-N-(3-(trifluoromethyl)phenyl)acetamide that affords (i) reduction in consumption of 2,2-dichloroacetyl chloride by about 5-15% in comparison to conventional wet method while affording similar yields of N-allyl-2,2-dichloro-N-(3-(trifluoromethyl)phenyl)acetamide; (ii) reduction in consumption of base by about 30-40%; and (iii) reduction in emission of HCl gas by about 60-90% in comparison to conventional dry methods greatly improving the process safety, while affording significant economic benefits.

## Claims

1. A process for preparing a N-substituted acetamide, the process comprising the steps of:
reacting an aniline or derivative thereof with a first amount of a chlorinated acyl chloride in presence of a mixture of an organic solvent and water for a first time period;
discarding the aqueous phase from the reaction mixture;
heating the reaction mixture at a reflux temperature;
effecting addition of a second amount of the chlorinated acyl chloride to the refluxing reaction mixture; and
continuing the reaction at the reflux temperature for a second time period to obtain a crude product mixture comprising N-substituted acetamide.

2. The process as claimed in claim 1, wherein the chlorinated acyl chloride is 2,2-dichloroacetyl chloride.

3. The process as claimed in claim 1, wherein the substituted aniline is N-allyl-3-(trifluoromethyl)aniline.

4. The process as claimed in claim 1, wherein the N-substituted acetamide is N-allyl-2,2-dichloro-N-(3-(trifluoromethyl)phenyl)acetamide.

5. The process as claimed in any of the preceding claims, wherein the process comprises the steps of:
reacting N-allyl-3-(trifluoromethyl) aniline with a first amount of 2,2-dichloroacetyl chloride in presence of a mixture of an organic solvent and water for a first time period, wherein N-allyl-3-(trifluoromethyl) aniline is reacted with the first amount of 2,2-dichloroacetyl chloride in a molar ratio ranging from 1:0.7 to 1:1.1;
discarding the aqueous phase from the reaction mixture;
heating the reaction mixture at a reflux temperature;
effecting addition of a second amount of 2,2-dichloroacetyl chloride to the refluxing reaction mixture; and
continuing the reaction at the reflux temperature for a second time period to obtain a crude product mixture comprising N-allyl-2,2-dichloro-N-(3-(trifluoromethyl) phenyl)acetamide.

6. The process as claimed in claim 5, wherein the step of reacting N-allyl-3-(trifluoromethyl)aniline with 2,2-dichloroacetyl chloride comprises: reacting N-allyl-3-(trifluoromethyl)aniline with a first amount of 2,2-dichloroacetyl chloride in presence of a mixture of an organic solvent and water under an inert atmosphere at a temperature ranging from 5°C to 30°C maintaining a pH between 5.0 and 11.0 for a first time period.

7. The process as claimed in claim 5, wherein the step of reacting N-allyl-3-(trifluoromethyl)aniline with 2,2-dichloroacetyl chloride comprises:
charging N-allyl-3-(trifluoromethyl)aniline and the mixture of organic solvent and water in a reaction vessel under the inert atmosphere to prepare a solution;
cooling the solution to a temperature ranging from 5°C to 20°C;
effecting addition of the first amount of 2,2-dichloroacetyl chloride to the cooled solution over a time period ranging from 60 minutes to 240 minutes maintaining temperature within 5°C and 20°C and pH within 5.0 to 11.0; and
stirring the reaction mixture for a time period ranging from 30 minutes to 120 minutes.

8. The process as claimed in claim 5, wherein the organic solvent is a water immiscible solvent inert to 2,2-dichloroacetyl chloride.

9. The process as claimed in claim 5, wherein the first time period ranges from 90 minutes to 360 minutes.

10. The process as claimed in claim 5, wherein the reflux temperature ranges from 80°C to 150°C.

11. The process as claimed in claim 5, wherein the step of heating the reaction mixture at a reflux temperature comprises heating the reaction mixture with azeotropic distillation.

12. The process as claimed in claim 5, wherein the step of effecting addition of the second amount of 2,2-dichloroacetyl chloride to the refluxing reaction mixture comprises adding 2,2-dichloroacetyl chloride in an amount such that the molar ratio of N-allyl-3-(trifluoromethyl)aniline to the second amount of 2,2-dichloroacetyl chloride ranges from 1:0.05 to 1:0.5.

13. The process as claimed in claim 5, wherein the second time period ranges from 30 minutes to 120 minutes.

14. A process for the preparation of Flurochloridone, the process comprising preparing an N-substituted acetamide according to the process of any one of claims 1-13.

## Patentansprüche

1. Ein Verfahren zur Herstellung eines N-substituierten Acetamids, wobei das Verfahren folgende Schritte umfasst:
das Reagieren eines Anilins oder eines Derivats davon mit einer ersten Menge eines chlorierten Acylchlorids in Anwesenheit einer Mischung aus einem organischen Lösungsmittel und Wasser über einen ersten Zeitraum;
das Wegschütten der wässerigen Phase aus der Reaktionsmischung;
das Erhitzen der Reaktionsmischung auf eine Rücklauftemperatur;
das Hinzufügen einer zweiten Menge des chlorierten Acylchlorids zur Rücklauf-Reaktionsmischung und
das Fortsetzen der Reaktion bei der Rücklauftemperatur über einen zweiten Zeitraum, um eine rohe Produktmischung zu erhalten, die N-substituiertes Acetamid umfasst.

2. Das Verfahren gemäß Anspruch 1, wobei das chlorierte Acylchlorid 2,2-Dichloracetylchlorid ist.

3. Das Verfahren gemäß Anspruch 1, wobei das substituierte Anilin N-Allyl-3-(trifluormethyl)anilin ist.

4. Das Verfahren gemäß Anspruch 1, wobei das N-substituierte Acetamid N-Allyl-2,2-dichlor-N-(3-(trifluormethyl)phenyl)acetamid ist.

5. Das Verfahren gemäß einem beliebigen der obigen Ansprüche, das folgende Schritte umfasst:
das Reagieren von N-Allyl-3-(trifluormethyl)anilin mit einer ersten Menge 2,2-Dichloracetylchlorid in Anwesenheit einer Mischung aus einem organischen Lösungsmittel und Wasser über einen ersten Zeitraum, wobei N-Allyl-3-(trifluormethyl)anilin mit der ersten Menge 2,2-Dichloracetylchlorid in einem Molverhältnis im Bereich von 1:0,7 bis 1:1,1 zur Reaktion gebracht wird;
das Wegschütten der wässerigen Phase aus der Reaktionsmischung;
das Erhitzen der Reaktionsmischung auf eine Rücklauftemperatur;
das Hinzufügen einer zweiten Menge 2,2-Dichloracetylchlorid zur Rücklauf-Reaktionsmischung und
das Fortsetzen der Reaktion bei der Rücklauftemperatur über einen zweiten Zeitraum, um eine rohe Produktmischung zu erhalten, die N-Allyl-2,2-dichlor-N-(3-(trifluormethyl)phenyl)acetamid umfasst.

6. Das Verfahren gemäß Anspruch 5, wobei der Schritt des Reagierens von N-Allyl-3-(trifluormethyl)anilin mit 2,2-Dichloracetylchlorid Folgendes umfasst: das Reagieren von N-Allyl-3-(trifluormethyl)anilin mit einer ersten Menge 2,2-Dichloracetylchlorid in Anwesenheit einer Mischung aus einem organischen Lösungsmittel und Wasser unter einer inerten Atmosphäre bei einer Temperatur im Bereich von 5°C bis 30°C, wobei ein pH-Wert zwischen 5,0 und 11,0 über einen ersten Zeitraum aufrechterhalten wird.

7. Das Verfahren gemäß Anspruch 5, wobei der Schritt des Reagierens von N-Allyl-3-(trifluormethyl)anilin mit 2,2-Dichloracetylchlorid Folgendes umfasst:
das Eintragen von N-Allyl-3-(trifluormethyl)anilin und der Mischung aus organischem Lösungsmittel und Wasser in ein Reaktionsgefäß unter der inerten Atmosphäre, um eine Lösung herzustellen,
das Kühlen der Lösung auf eine Temperatur im Bereich von 5°C bis 20°C,
das Hinzufügen der ersten Menge 2,2-Dichloracetylchlorid zur gekühlten Lösung über einen Zeitraum im Bereich von 60 Minuten bis 240 Minuten, wobei die Temperatur innerhalb von 5°C und 20°C und der pH-Wert innerhalb von 5,0 bis 11,0 gehalten wird; und
das Rühren der Reaktionsmischung über einen Zeitraum im Bereich von 30 Minuten bis 120 Minuten.

8. Das Verfahren gemäß Anspruch 5, wobei das organische Lösungsmittel ein nicht mit Wasser mischbares Lösungsmittel ist, das gegenüber 2,2-Dichloracetylchlorid inert ist.

9. Das Verfahren gemäß Anspruch 5, wobei der erste Zeitraum im Bereich von 90 Minuten bis 360 Minuten liegt.

10. Das Verfahren gemäß Anspruch 5, wobei die Rücklauftemperatur im Bereich von 80°C bis 150°C liegt.

11. Das Verfahren gemäß Anspruch 5, wobei der Schritt des Erhitzens der Reaktionsmischung auf eine Rücklauftemperatur das Erhitzen der Reaktionsmischung mit azeotroper Destillation umfasst.

12. Das Verfahren gemäß Anspruch 5, wobei der Schritt des Hinzufügens der zweiten Menge 2,2-Dichloracetylchlorid zur Rücklauf-Reaktionsmischung das Hinzufügen von 2,2-Dichloracetylchlorid in einer solchen Menge umfasst, dass das Molverhältnis von N-Allyl-3-(trifluormethyl)anilin zur zweiten Menge 2,2-Dichloracetylchlorid im Bereich von 1:0,05 bis 1:0,5 liegt.

13. Das Verfahren gemäß Anspruch 5, wobei der zweite Zeitraum im Bereich von 30 Minuten bis 120 Minuten liegt.

14. Ein Verfahren zur Herstellung von Fluorchloridon, wobei das Verfahren das Herstellen eines N-substituierten Acetamids nach dem Verfahren gemäß einem beliebigen der Ansprüche 1-13 umfasst.

## Revendications

1. Procédé de préparation d'un acétamide N-substitué, le procédé comprenant les étapes suivantes :
faire réagir une aniline ou un dérivé de celle-ci avec une première quantité d'un chlorure d'acyle chloré en présence d'un mélange d'un solvant organique et d'eau pendant une premère période de temps ;
éliminer la phase aqueuse du mélange réactionnel ;
chauffer le mélange réactionnel à une température de reflux ;
effectuer l'addition d'une deuxième quantité du chlorure d'acyle chloré au mélange réactionnel sous reflux ; et
poursuivre la réaction à la température de reflux pendant une deuxième période de temps pour obtenir un mélange de produit brut comprenant de l'acétamide N-substitué.

2. Procédé selon la revendication 1, où le chlorure d'acyle chloré est le chlorure de 2,2-dichloroacétyle.

3. Procédé selon la revendication 1, où l'aniline substituée est la N-allyl-3-(trifluorométhyl)aniline.

4. Procédé selon la revendication 1, où l'acétamide N-substitué est le N-allyl-2,2-dichloro-N-(3-(trifluorométhyl)phényl)acétamide.

5. Procédé selon l'une quelconque des revendications précédentes, où le procédé comprend les étapes suivantes :
faire réagir la N-allyl-3-(trifluorométhyl)aniline avec une première quantité de chlorure de 2,2-dichloroacétyle en présence d'un mélange d'un solvant organique et d'eau pendant une première période de temps, où la N-allyl -3-(trifluorométhyl)aniline réagit avec la première quantité de chlorure de 2,2-dichloroacétyle dans un rapport molaire compris entre 1:0,7 et 1:1,1 ;
éliminer la phase aqueuse du mélange réactionnel ;
chauffer le mélange réactionnel à une température de reflux ;
effectuer l'addition d'une deuxième quantité de chlorure de 2,2-dichloroacétyle au mélange réactionnel sous reflux ; et
poursuivre la réaction à la température de reflux pendant une deuxième période de temps pour obtenir un mélange de produit brut comprenant du N-allyl-2,2-dichloro-N-(3-(trifluorométhyl)phényl)acétamide.

6. Procédé selon la revendication 5, où l'étape consistant à faire réagir la N-allyl-3-(trifluorométhyl)aniline avec le chlorure de 2,2-dichloroacétyle comprend : la réaction de la N-allyl-3-(trifluorométhyl)aniline avec une première quantité de chlorure de 2,2-dichloroacétyle en présence d'un mélange d'un solvant organique et d'eau sous une atmosphère inerte à une température comprise entre 5 °C et 30 °C en maintenant un pH compris entre 5,0 et 11,0 pendant une première période.

7. Procédé selon la revendication 5, où l'étape de réaction de la N-allyl-3-(trifluorométhyl) aniline avec le chlorure de 2,2-dichloroacétyle comprend :
le chargement de la N-allyl-3-(trifluorométhyl)aniline et du mélange de solvant organique et d'eau dans un récipient de réaction sous atmosphère inerte pour préparer une solution ;
le refroidissement de la solution à une température comprise entre 5 °C et 20 °C ;
effectuer l'addition de la première quantité de chlorure de 2,2-dichloroacétyle à la solution refroidie pendant une période de temps comprise entre 60 minutes et 240 minutes, en maintenant la température entre 5 °C et 20 °C et le pH entre 5,0 et 11,0 ; et
agiter le mélange réactionnel pendant une période de temps comprise entre 30 minutes et 120 minutes.

8. Procédé selon la revendication 5, où le solvant organique est un solvant non miscible à l'eau et inerte vis-à-vis du chlorure de 2,2-dichloroacétyle.

9. Procédé selon la revendication 5, où la première période de temps est comprise entre 90 minutes et 360 minutes.

10. Procédé selon la revendication 5, où la température de reflux est comprise entre 80 °C et 150 °C.

11. Procédé selon la revendication 5, où l'étape consistant à chauffer le mélange réactionnel à une température de reflux comprend le chauffage du mélange réactionnel par distillation azéotropique.

12. Procédé selon la revendication 5, où l'étape consistant à effectuer l'addition de la deuxième quantité de chlorure de 2,2-dichloroacétyle au mélange réactionnel sous reflux comprend l'addition de chlorure de 2,2-dichloroacétyle en une quantité telle que le rapport molaire de la N-allyl-3-(trifluorométhyl)aniline à la deuxième quantité de chlorure de 2,2-dichloroacétyle soit compris entre 1:0,05 et 1:0,5.

13. Procédé selon la revendication 5, où la deuxième période de temps est comprise entre 30 minutes et 120 minutes.

14. Procédé de préparation de la fluorochloridone, le procédé comprenant la préparation d'un acétamide N-substitué selon le procédé de l'une quelconque des revendications 1 à 13.
